# EUROPEAN PATENT SPECIFICATION

(11) **EP 2 691 535 B1**
(45) Date of publication and mention of the grant of the patent: **04.01.2017**
(21) Application number: 12712077.2
(22) Date of filing: 30.03.2012
(51) Int. Cl.: C12Q 1/68

(54) **CANCER MARKERS**
KREBSMARKER
MARQUEURS DU CANCER

(30) Priority: 31.03.2011 EP 11160657
(43) Date of publication of application: 05.02.2014
(73) Proprietor: Queen Mary and Westfield College University of London, London E1 4NS (GB)
(72) Inventor: LORINCZ, Attila, London EC1M 6BQ (GB)
(74) Representative: CH Kilger Anwaltspartnerschaft mbB
(86) International application number: PCT/EP2012/001477
(87) International publication number: WO 2012/130478

(56) References cited:
- WO-A2-2006/008128
- J YU ET AL: "The neuronal repellent SLIT2 is a target for repression by EZH2 in prostate cancer", ONCOGENE, vol. 29, no. 39, 30 September 2010 (2010-09-30), pages 5370-5380, XP55026622, ISSN: 0950-9232, DOI: 10.1038/onc.2010.269
- S. S. Y. TEOH ET AL: "Maspin (SERPINB5) Is an Obligate Intracellular Serpin", JOURNAL OF BIOLOGICAL CHEMISTRY, vol. 285, no. 14, 2 April 2010 (2010-04-02), pages 10862-10869, XP55026625, ISSN: 0021-9258, DOI: 10.1074/jbc.M109.073171
- I KOGAN-SAKIN ET AL: "Mutant p53R175H upregulates Twist1 expression and promotes epithelial-mesenchymal transition in immortalized prostate cells", CELL DEATH AND DIFFERENTIATION, vol. 18, no. 2, 1 February 2011 (2011-02-01), pages 271-281, XP55026630, ISSN: 1350-9047, DOI: 10.1038/cdd.2010.94
- WANG YIPENG ET AL: "Survey of differentially methylated promoters in prostate cancer cell lines.", NEOPLASIA (NEW YORK, N.Y.) AUG 2005 LNKD- PUBMED:16207477, vol. 7, no. 8, August 2005 (2005-08), pages 748-760, XP002675723, ISSN: 1522-8002
- MORA-GARCÍA MARÍA DE LOURDES ET AL: "Up-regulation of HLA class-I antigen expression and antigen-specific CTL response in cervical cancer cells by the demethylating agent hydralazine and the histone deacetylase inhibitor valproic acid", JOURNAL OF TRANSLATIONAL MEDICINE, BIOMED CENTRAL, LONDON, GB, vol. 4, no. 1, 27 December 2006 (2006-12-27), page 55, XP021024919, ISSN: 1479-5876, DOI: 10.1186/1479-5876-4-55
- STEINER ISABEL ET AL: "Gene promoter methylation and its potential relevance in early prostate cancer diagnosis.", PATHOBIOLOGY : JOURNAL OF IMMUNOPATHOLOGY, MOLECULAR AND CELLULAR BIOLOGY 2010 LNKD- PUBMED:21116117, vol. 77, no. 5, 2010, pages 260-266, XP9159220, ISSN: 1423-0291
- WOONBOK CHUNG ET AL: "Identification of Novel Tumor Markers in Prostate, Colon and Breast Cancer by Unbiased Methylation Profiling", PLOS ONE, vol. 3§§§§§§§§§§§§§§§§§§§§§§§§§§§§§§§§§§§§§§§§§ §§§§§§§§, no. 4, 1 January 2008 (2008-01-01), page E2079, XP55026709, ISSN: 1932-6203, DOI: 10.1371/journal.pone.0002079
- MCEVOY C R E ET AL: "Frequency and genetic basis of MHC, beta-2-microglobulin and MEMO-1 loss of heterozygosity in sporadic breast cancer.", TISSUE ANTIGENS SEP 2002 LNKD- PUBMED:12445306, vol. 60, no. 3, September 2002 (2002-09), pages 235-243, XP002678943, ISSN: 0001-2815

### SEQUENCE LISTING

<110> Queen Mary and westfield college, university of London
<120> Prostate Cancer Markers
<130> B051-0085WO01
<150> EP 11 16 0657.0
   <151> 2011-03-31
<160> 7
<170> PatentIn version 3.5
<210> 1
   <211> 121
   <212> DNA
   <213> Homo sapiens
<400> 1
   ggagaaggtg gagactgagc tccagggcgt gtgcgacacc gtgctgggcc tgctggacag 60
   ccacctcatc aaggaggccg gggacgccga gagccgggtc ttctacctga agatgaaggg 120
   t 121
<210> 2
   <211> 112
   <212> DNA
   <213> Homo sapiens
<400> 2
   agatcccctc ttctgtcttg taccttcgcc actggcatcg gatttgcaga agcgtgcgtg 60
   ggatcagagg accgccctcc ccacaacaac cggcccctgc atcttagcag cc 112
<210> 3
   <211> 71
   <212> DNA
   <213> Homo sapiens
<400> 3
   caagaggctt gagtaggaga ggagtgccgc cgaggcgggg cggggcgggg cgtggagctg 60
   ggctggcagt g 71
<210> 4
   <211> 98
   <212> DNA
   <213> Homo sapiens
<400> 4
   tcctcctgct ctctcctccg cgggccgcat cgcccgggcc ggcgccgcgc gcgggggaag 60
   ctggcgggct gaggcgcccc gctcttctcc tctgcccc 98
<210> 5
   <211> 95
   <212> DNA
   <213> Homo sapiens
<400> 5
   gggctagggc taggcaggct gtgcggttgg gcggggccct gtgccccact gcggagtgcg 60
   ggtcgggaag cggagagaga agcagctgtg taatc 95
<210> 6
   <211> 41
   <212> DNA
   <213> Homo sapiens
<400> 6
   gggccctggc cctgacccag acctgggcgg gtgagtgcgg g 41
<210> 7
   <211> 116
   <212> DNA
   <213> Homo sapiens
<400> 7
   ccttctcagt caaagacatc ctaaacctgg aacagcagca gcgcagcctg gctgccgccg 60
   gagagctctc tgcccgcctg gaggcgaccc tggcgccctc ctcctgcatg ctggcc 116

## Description

### FIELD OF THE INVENTION

The present invention is in the field of biology and chemistry. In particular, the invention is in the field of molecular biology. More particular, the invention relates to the analysis of the methylation status of genomic regions. Most particularly, the disclosure is in the field of diagnosis and/or prognosis of prostate and breast cancer but also of cancer in general.

### BACKGROUND

Reversible methylation of cytosines is a major epigenetic modification in multicellular organisms and is found in many human diseases including cancer. Cancer epigenomes are found to be globally hypomethylated with promoter-specific hypermethylations. Furthermore, cytosine methylation results in transcriptional repression, which, in the case of tumour suppressor genes, apoptotic genes, DNA repair genes and factors controlling cell cycle check points, leads to tumour progression.

Prostate cancer (PCa) is the third most common cause of male cancer deaths in developed countries. Diagnosed at an early stage PCa is a curable disease. Clinical management approaches depend on the extent and severity of the cancer and in early stage low grade cancer may consist mostly of watchful waiting (also called expectant management) whereas in advanced or aggressive cancers treatments can include radical prostatectomy, hormone or radiation therapy. Nevertheless, because of the cancer's mostly unpredictable outcome patients are often treated without clear benefit and there is a recognized major problem of substantial overtreatment in many countries.

Prostate specific antigen (PSA) is used as a biomarker to screen men for potential tumour developments. However, low specificity and moderate sensitivity lead to many falsely diagnosed prostate cancers. In particular, elevated PSA can also result from an inflammation or precedent transrectal ultrasound, i.e. disclosure within the state of the art lacks an unequivocal diagnosis of PCa.

It is therefore clear that there has been and remains today a long standing need for accurate and reliable prognostic markers.

Recent years have brought a marked extension of our understanding of the somatic basis of prostate cancer. With one to three mutations per megabase the mutation frequency is similar to that observed in acute myeloid leukemia and invasive breast cancer (IBC) and lies within the lower range of cancer mutations. Based on the frequency and the fact that primarily a diverse array of genes are affected the main genomic alterations appear to be genomic rearrangements and changes in the epigenetic structure of the DNA.

Aberrant DNA methylation plays an important role in prostate cancer development and seems to be one of the earliest events in tumorigenesis. The most prominent differentially methylated gene in prostate cancer is glutathione S-transferase pi 1 (GSTP1).

Other genes with changes in promoter methylation include multidrug resistance protein 1 (MDR1), O-6-methylguanine-DNA methyltransferase (MGMT), Ras association domain family member 1 (RASSF1), retinoic acid receptor beta (RARB), adenomatous polyposis coli (APC), androgen receptor (AR), cyclin-dependent kinase inhibitor 2A (CDKN2A), E-cadherin (CDH1) and CD44, but some of these genes show inconsistent methylation levels and sometimes no DNA methylation in different studies.

Breast cancer (malignant breast neoplasm) is cancer originating from breast tissue, most commonly from the inner lining of milk ducts or the lobules that supply the ducts with milk. Cancers originating from ducts are known as ductal carcinomas; those originating from lobules are known as lobular carcinomas.

The size, stage, rate of growth, and other characteristics of the tumor determine the kinds of treatment. Treatment may include surgery, drugs (hormonal therapy and chemotherapy), radiation and/or immunotherapy. Surgical removal of the tumor provides the single largest benefit, with surgery alone being capable of producing a cure in many cases. To somewhat increase the likelihood of long-term disease-free survival, several chemotherapy regimens are commonly given in addition to surgery. Most forms of chemotherapy kill cells that are dividing rapidly anywhere in the body, and as a result cause temporary hair loss and digestive disturbances and occasionally cardiotoxicity. Radiation may be added to kill any cancer cells in the breast that were missed by the surgery, which usually extends survival somewhat, although radiation exposure to the heart may cause heart failure in the future. Some breast cancers are sensitive to hormones such as estrogen and/or progesterone, which makes it possible to treat them by blocking the effects of these hormones for example by use of anti-estrogens like tamoxifen or aromatase inhibitors that block the body's synthesis of estrogen.

Prognosis and survival rate varies greatly depending on cancer type and staging.

Also for IBC there is need for additional reliable diagnostic and prognostic markers.

### SUMMARY OF THE INVENTION

The description supplies a solution to the above-mentioned problem, by providing for a method for diagnosis and/or prognosis of cancer, comprising the steps of (a) analyzing in a sample of a subject the DNA methylation status of a genomic region of at least one member of the group of, (i) SFN according to SEQ ID NO. 1, (ii) SLIT2 according to SEQ ID NO. 2, (iii) SERPINB5 according to SEQ ID NO. 3 (iv) or TWIST1; wherein, if (i) SFN shows a methylation cut-off value of above 80 % and/or, (ii) SLIT2 shows a methylation cut-off value of above 45 % and/or, SERPINB5 shows a methylation cut-off value of above 70 %, the sample is categorized as a sample from a patient with cancer with a poor prognosis.

The description also relates to respective nucleic acids, compositions and kits.

### DEFINITIONS

The following definitions are provided for specific terms which are used in the following.

As used herein, the term "amplified", when applied to a nucleic acid sequence, refers to a process whereby one or more copies of a particular nucleic acid sequence is generated from a nucleic acid template sequence, preferably by the method of polymerase chain reaction. The reaction mix comprises dNTPs (each of the four deoxynucleotides dATP, dCTP, dGTP, and dTTP), primers, buffers, DNA polymerase, and nucleic acid template. The PCR reaction can comprise (a) providing a "primer pair" wherein a first primer contains a sequence complementary to the sense strand of the target nucleic acid sequence and primes the synthesis of a complementary second DNA strand, and a second primer contains a sequence complementary to the antisense strand of the target nucleic acid sequence and primes the synthesis of a complementary DNA strand of the antisense strand, and (b) amplifying the nucleic acid template sequence employing a nucleic acid polymerase. Usually, a Taq polymerase is used to amplify a nucleic acid template in PCR reaction. Other methods of amplification include, but are not limited to, ligase chain reaction (LCR), polynucleotide-specific based amplification, or any other method known in the art.

As used herein, the term "biomarker" refers to (a) a genomic region that is differentially methylated, or (b) a gene that is differentially expressed, or (c) a mutation of a DNA sequence or single-nucleotide polymorphism (SNP) that can be associated with subjects having cancer or a stage of cancer compared with those not having cancer.

As used herein, the term "composition" refers to any mixture. It can be a solution, a suspension, liquid, powder, a paste, aqueous, non-aqueous or any combination thereof.

The term "CpG position" as used herein refers to regions of DNA where a cytosine nucleotide is located at the 5' adjacent position to a guanine nucleotide in the linear sequence of bases along its length. "CpG" is shorthand for "C-phosphate-G", that is, cytosine and guanine separated by a phosphate, which links the two nucleosides together in DNA. Cytosines in CpG dinucleotides can be methylated to form 5-methylcytosine.

As used herein, the term "diagnosis" refers to the identification of cancer (for example prostate cancer (PCa) or invasive breast cancer (IBC) at any stage of its development, and also includes the determination of predisposition of a subject to develop the disease.

The term "prognosis" as used herein refers to a prediction of the probable course and outcome of a clinical condition or disease. A prognosis of a patient is usually made by evaluating factors, markers, and/or symptoms of a disease that are indicative of a favourable or unfavourable course or outcome of the disease.

The phrase "determining the prognosis" as used herein refers to the process by which the skilled artisan can predict the course or outcome of a condition in a patient. The term "prognosis" does not refer to the ability to predict the course or outcome of a condition with 100 % accuracy. Instead, the skilled artisan will understand that the term "prognosis" refers to an increased probability that a certain course or outcome will occur; that is, that a course or outcome is more likely to occur in a patient exhibiting a given condition, when compared to those individuals not exhibiting the condition. A prognosis may be expressed as the amount of time a patient can be expected to survive. Alternatively, a prognosis may refer to the likelihood that the disease goes into remission or to the amount of time the disease can be expected to remain in remission. Prognosis can be expressed in various ways; for example prognosis can be expressed as a percent chance that a patient will survive after one year, five years, ten years or the like. Alternatively, prognosis may be expressed as the number of years, on average, that a patient can expect to survive as a result of a condition or disease. The prognosis of a patient may be considered as an expression of relativism, with many factors effecting the ultimate outcome. For example, for patients with certain conditions, prognosis can be appropriately expressed as the likelihood that a condition may be treatable or curable, or the likelihood that a disease will go into remission, whereas for patients with more severe conditions prognosis may be more appropriately expressed as likelihood of survival for a specified period of time.

As used herein, the term "differential methylation" refers to a difference in the level of DNA/cytosine methylation in a cancer positive sample as compared with the level of DNA methylation in a cancer negative sample. It may also refer to the difference in levels between patients that have recurrence of cancer after surgery versus patients who not have recurrence. Differential methylation and specific levels or patterns of DNA methylation can be used as prognostic and predictive biomarkers once the correct cut-off or predictive characteristics have been defined. The "DNA methylation status" is interchangeable with the term "DNA methylation level" and may be assessed by determining the ratio of methylated and non-methylated DNA for a genomic region or a portion thereof and is quoted in percentage. The methylation status is classified herein as either increased or decreased and may relate to a person with recurrence of cancer as compared to a control person who did experience a recurrence during a similar observation period. Alternatively DNA methylation may be either increased or decreased in given marker genes in a person with a pathologically diagnosed high grade cancer that is known to be of higher risk of progression as compared to a person with a low grade cancer who is likely to have a favourable outcome.

Herein, a "cut-off value" is defined as follows: a specific DNA methylation level above which results are regarded as positive (or negative for a gene with a reverse association) versus when the methylation level is below the cut-off the results are regarded as negative (or positive for a gene with reverse association). To account for biological variability that is known to be typical of all living biological systems such as humans or other organisms it is reasonable to consider ranges of values and thus all cut-off values herein may vary by plus minus 15 %, plus minus 10 % or preferably only plus minus 5 %. This also depends on the experimental set-up.

The term "analyzing the methylation status" as used herein, relates to the means and methods useful for assessing the methylation status. Useful methods are bisulphite-based methods, such as bisulphite-based mass spectrometry or bisulphite-based sequencing methods.

The term "genomic region specific primers" as used herein refers to a primer pair complementary to a sequence in the vicinity of a genomic region according to the invention, which can be produced by methods of amplification of double-stranded DNA complementary to a genomic region of the invention.

The term "genomic region specific probe" as used herein refers to a probe that selectively hybridizes to a DNA product of a genomic region. In one embodiment a genomic region specific probe can be a probe labelled, for example, with a fluorophore and a quencher, such as a TaqMan® probe or a Molecular Beacons probes.

As used herein, the terms "hybridizing to" and "hybridization" are interchangeably used with the term "specific for" and refer to the sequence-specific non-covalent binding interactions with a complementary nucleic acid, for example, interactions between a target nucleic acid sequence and a target specific nucleic acid primer or probe. In a preferred embodiment a nucleic acid, which hybridizes, is one which hybridizes with a selectivity of greater than 70 %, greater than 80 %, greater than 90 % and most preferably of 100 % (i.e. cross hybridization with other DNA species preferably occurs at less than 30 %, less than 20 %, less than 10 %). As would be understood to a person skilled in the art, a nucleic acid, which "hybridizes" to the DNA product of a genomic region of the invention, can be determined taking into account the length and composition.

As used herein, "isolated" when used in reference to a nucleic acid means that a naturally occurring sequence has been removed from its normal cellular (e.g. chromosomal) environment or is synthesised in a non-natural environment (e.g. artificially synthesised). Thus, an "isolated" sequence may be in a cell-free solution or placed in a different cellular environment.

As used herein, a "kit" is a packaged combination optionally including instructions for use of the combination and/or other reactions and components for such use.

As used herein, "nucleic acid(s)" or "nucleic acid molecule" generally refers to any ribonucleic acid or deoxyribonucleic acid, which may be unmodified or modified DNA or RNA. "Nucleic acids" include, without limitation, single- and double-stranded nucleic acids. As used herein, the term "nucleic acid(s)" also includes DNA as described above that contain one or more modified bases. Thus, DNA with backbones modified for stability or for other reasons are "nucleic acids". The term "nucleic acid(s)" as it is used herein embraces such chemically, enzymatically or metabolically modified forms of nucleic acids, as well as the chemical forms of DNA characteristic of viruses and cells, including for example, simple and complex cells.

The term "primer" as used herein refers to a nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. For example, for diagnostic applications, depending on the complexity of the target sequence, the nucleic acid primer typically contains 15-25 or more nucleotides, although it may contain fewer nucleotides.

As used herein, the term "probe" means nucleic acid and analogs thereof and refers to a range of chemical species that recognise polynucleotide target sequences through hydrogen bonding interactions with the nucleotide bases of the target sequences. The probe or the target sequences may be single- or double-stranded DNA. A probe is at least 8 nucleotides in length and less than the length of a complete polynucleotide target sequence. A probe may be 10, 20, 30, 50, 75, 100, 150, 200, 250, 400, 500 and up to 10,000 nucleotides in length. Probes can include nucleic acids modified so as to have one or more tags which are detectable by fluorescence, chemiluminescence and the like ("labelled probe"). The labelled probe can also be modified so as to have both one or more detectable tags and one or more quencher molecules, for example Taqman® and Molecular Beacon® probes. The nucleic acid and analogs thereof may be DNA, or analogs of DNA, commonly referred to as antisense oligomers or antisense nucleic acid. Such DNA analogs comprise but are not limited to 2-'O-alkyl sugar modifications, methylphosphonate, phosphorothiate, phosphorodithioate, formacetal, 3'-thioformacetal, sulfone, sulfamate, and nitroxide backbone modifications, and analogs wherein the base moieties have been modified. In addition, analogs of oligomers may be polymers in which the sugar moiety has been modified or replaced by another suitable moiety, resulting in polymers which include, but are not limited to, morpholino analogs and peptide nucleic acid (PNA) analogs (Egholm, et al. Peptide Nucleic Acids (PNA)-Oligonucleotide Analogues with an Achiral Peptide Backbone, (1992)).

The term "sample" is used herein to refer to tissue per se, cancer tissue, potential cancer tissue, prostate or breast tissue, blood, urine, semen, prostatic secretions, milk, breast exudates, needle aspirations or isolated prostate or breast cells, cells originating from a subject, preferably from prostate tissue, breast tissue, prostatic secretions, breast secretions or isolated prostate cells or breast cells, most preferably to prostate tissue or breast tissue.

The term "bisulphite sequencing" refers to a method well-known to the person skilled in the art comprising the steps of (a) treating the DNA of interest with bisulphite, thereby converting non-methylated cytosines to uracils and leaving methylated cytosines unaffected and (b) sequencing the treated DNA, wherein the existence of a methylated cytosine is revealed by the detection of a non-converted cytosine and the absence of a methylated cytosine is revealed by the detection of an uracil.

As used herein, "stringent conditions for hybridization" are known to those skilled in the art and can be found in Current Protocols in Molecular Biology, John Wiley & Sons, N.Y., 6.3.1-6.3.6, 1991. Stringent conditions are defined as equivalent to hybridization in 6X sodium chloride/sodium citrate (SSC) at 45 °C, followed by a wash in 0.2 X SSC, 0.1 % SDS at 65 °C.

As used herein, the terms "subject" and "patient" are used interchangeably to refer to a human or an animal (e.g., a mammal, a fish, an amphibian, a reptile, a bird and an insect). In a specific embodiment, a subject is a mammal (e.g., a non-human mammal and a human). In another embodiment, a subject is a pet (e.g., a dog, a cat, a guinea pig, a monkey and a bird), a farm animal (e.g., a horse, a cow, a pig, a goat and a chicken) or a laboratory animal (e.g., a mouse and a rat). In another embodiment, a subject is a primate (e.g., a chimpanzee and a human). In another embodiment, a subject is a human. In another embodiment, the subject is a male human or a female human.

As used herein, the term "in the vicinity of a genomic region" refers to a position outside or within said genomic region. As would be understood by a person skilled in the art the position may have a distance up to 1000 nt, preferably up to 500 nt, more preferably up to 200 nt from the 5' or 3' end of the genomic region. Even more preferably the position is located at the 5' or 3' end of said genomic region. In another embodiment of the invention the position is within said genomic region.

### DETAILED DESCRIPTION OF THE INVENTION

The invention as disclosed herein identifies genomic regions that are useful in diagnosing aggressive cancer. These are also prognostic markers.

By definition, the identified genomic regions are biomarkers for aggressive cancer. In order to use these genomic regions (as biomarkers), the invention teaches the analysis of the DNA methylation status of said genomic regions. The invention further encompasses genomic region specific nucleic acids. The invention further contemplates the use of said genomic region specific nucleic acids to analyze the methylation status of a genomic region, either directly or indirectly by methods known to the skilled person and explained herein. The invention further discloses a composition and kit comprising said nucleic acids for the diagnosis of PCa or IBC. The inventors found genomic regions that are subject to an aberrant methylation status. Tumour associations were found. Therefore, the invention teaches the analysis of those genomic regions that are differentially methylated in samples from patients having cancer. Superior to current diagnostic methods, the invention discloses genomic regions, wherein most astonishingly a combination of up to four genomic regions works very well.

Hence, the disclosure relates to a method for diagnosis and/or prognosis of cancer, comprising the steps of (a) analyzing in a sample of a subject the DNA methylation status of a genomic region of at least one member of the group of, (i) SFN according to SEQ ID NO. 1, (ii) SLIT2 according to SEQ ID NO. 2, (iii) SERPINB5 according to SEQ ID NO. 3; wherein, if (i) SFN shows a methylation cut-off value of above 80 % and/or, (ii) SLIT2 shows a methylation cut-off value of above 45 % and/or, SERPBINB5 shows a methylation cut-off value of above 70 %, the sample is categorized as a sample from a patient with a cancer of poor prognosis.

| **Region name** | *Genomic regions from 5'end of Fprimer to 5' end of R primer* |
|---|---|
| SFN (SEQ ID NO. 1) | |
| SLIT2 (SEQ ID NO. 2) | |
| SERPINB5 (SEQ ID NO. 3) | |
| TWIST 1 (SEQ ID NO. 4) | |

Accession numbers are shown below:

| Gene | Accession number NCBI Reference Sequence | Accession number UCSC human gene sorter |
|---|---|---|
| SFN | NM_006142 (mRNA) | uc00lbnc.1 |
| SLIT2 | NT_006316.16 | uc003gpr.1 |
| SERPINB5 | NT_025028.14 | uc002liz.3 |
| TWIST 1 | NM_000474.3 (mRNA) | uc003sum.2 |

Accordingly, the invention relates to a method for prognosis of prostate cancer (PCa).

The invention relates to a method for prognosis of prostate cancer comprising the steps of analyzing in a sample of a subject the DNA methylation status of the following genomic regions, (i) SFN according to SEQ ID NO. 1, (ii) SLIT2 according to SEQ ID NO.2 and (iii) SERPINB5 according to SEQ ID NO.3, wherein, if (i) SFN shows a methylation cut-off value of above 80 % and/or, (ii) SLIT2 shows a methylation cut-off value of above 45 % and/or, (iii) SERPBINB5 shows a methylation cut-off value of above 70 %, and /or (iv) TWIS1 shows a methylation value below 15 %, preferably below 10 %, and most preferably below 5 %, the sample is categorized as a sample from a patient with cancer or with a poor prognosis. In this embodiment all four regions are analyzed at their respect cut offs and methylation scales and the data are used in an additive risk formula to produce only one clinical cut-off that is used to categorize a sample as of poor prognosis or not.

In a preferred embodiment of the disclosure diagnosis of cancer occurs prior to the manifestation of symptoms. Subjects with a higher risk of developing aggressive disease are of particular concern. The diagnostic method of the disclosure also allows confirmation of cancer in a subject suspected of having cancer or aggressive cancer.

The method of the disclosure is particularly useful for early diagnosis of prostate cancer (PCa) or breast cancer (IBC). The method is useful for further diagnosing patients having an identified prostate mass or symptoms associated with prostate cancer, e.g. abnormally high levels of PSA. The method is also useful as a follow-up test to women who have been diagnosed with breast cancer or who have abnormal mammograms and can provide biopsies, tissues, aspirate, or other tissue fluids to assess the methylation status of the indicated genes. The method of the present disclosure can further be of particular use with patients having an enhanced risk of developing prostate or breast cancer (e.g., patients having a familial history of prostate or breast cancer and patients identified as having mutant oncogenes or other risk factors). The method of the present disclosure may further be of particular use in monitoring the efficacy of treatment of a prostate or breast cancer patient (e.g. the efficacy of chemotherapy).

In one embodiment of the method of the disclosure the sample comprises cells obtained from a patient. The cells may be found in a prostate tissue sample or a breast sample collected, for example, by a tissue biopsy or histology section. In another embodiment, the patient sample is a prostate- or breast-associated body fluid. Such fluids include, for example, blood fluids, lymph, urine, prostatic fluid, semen, breast aspirates, or exudates, or milk and may include isolated cancer cells separated from heterogeneous human clinical specimens by use of separation and purification methods such as immune selections, flow sorting, or other methods to enrich for the desired cancer cells. The DNA can be isolated from the sample by means of cell disruption or cell lysis by sonication and/or enzymatic digestion, treatment with detergents to remove membrane lipids and precipitation of the DNA using alcohol. Cell-free DNA can also be collected and purified from fluids that contain few if any cells, such as for example serum. Then the DNA can be forwarded to the analysis method.

In order to analyze the methylation level status of a genomic region, conventional quantitative or semi-quantitative technologies can be used.

First, the extracted DNA of interest may be enriched, for example by methylated DNA immunoprecipitation (MeDIP). Then, the methylation status of the DNA can be analyzed either directly or after bisulphite treatment.

In one embodiment, bisulphite-based approaches are used to preserve the methylation information. Therefore, the DNA is treated with bisulphite, thereby converting non-methylated cytosine residues into uracil while methylated cytosines are left unaffected. This selective conversion makes the methylation easily detectable and quantifiable by classical methods that reveal the existence or absence of DNA (cytosine) methylation of the DNA of interest. The DNA of interest may be amplified before the detection if necessary. Such detection can be done by mass spectrometry. Preferably, the DNA of interest is sequenced. Suitable sequencing methods are direct sequencing and pyrosequencing. In another embodiment of the invention the DNA of interest is detected by a genomic region specific probe that is quantitatively selective for that sequence in which a cytosine was either converted or not converted. Other techniques that can be applied after bisulphite treatment are methylation-sensitive single-strand conformation analysis (MS-SSCA), high resolution melting analysis (HRM), methylation-sensitive single-nucleotide primer extension (MS-SnuPE) and base-specific cleavage, and epiTYPER methods. A further method is shown in "Sensitive digital quantification of DNA methylation in clinical samples" Nat Biotechnol. 2009 September; 27(9): 858-863. It is also referred to as Methyl-BEAMing.

In an alternative embodiment the methylation status of the DNA is analyzed without bisulphite treatment, such as cleavage by enzymes that are sensitive to DNA methylation levels followed by methylation-specific PCR or by the use of a genomic region specific probe that are selective for that sequence in which a cytosine is either methylated or non-methylated as indicated by the cleavage.

To translate the raw data generated by the detection assay (e.g. a nucleotide sequence) into data of predictive value for a clinician, a computer-based risk analysis program can be used.

The profile data may be prepared in a format suitable for interpretation by a treating clinician. For example, rather than providing raw nucleotide sequence data or methylation status, the prepared format may represent a diagnosis or risk assessment (e.g. likelihood of cancer being present or the subtype of cancer) for the subject, along with recommendations for particular treatment options.

In some embodiments, the results are used in a clinical setting to determine the further course of action. In other embodiments, the results are used to determine a treatment course of action (e.g., choice of therapies or watchful waiting).

Preferably the methylation status of a further genomic region and/or a further biomarker is analyzed which may be selected from the group of:
APC according to SEQ ID NO. 5.
HLAa according to SEQ ID NO. 6 and/or
NKX2-5 according to SEQ ID NO. 7

The sample is categorized as outlined above and the cut-offs are:
3 % for APC according to SEQ ID NO.5,
35 % for HLAa according to SEQ ID NO. 6 and/or
5 % NKX2-5 according to SEQ ID NO. 7

Patients with specimens that have methylation percentage above the cut-offs for APC and HLAa are regarded as at increased risk for aggressive cancer and conversely where patients with methylation percentages below the cut-off of NKX2-5 are regarded as of higher risk for aggressive cancer.

| | |
|---|---|
| APC (SEQ ID NO. 5) | |
| HLAa (SEQ ID NO. 6) | |
| NKX2-5 (SEQ ID NO. 7) | |

Accession numbers are shown below:

| Gene | Accession number NCBI Reference Sequence | Accession number UCSC human gene sorter |
|---|---|---|
| APC | NT_034772.6 | uc003kpy.3 |
| HLAa | NT_113891.2 | uc003nol.2 |
| NKX2-5 | NM_001166176.1 (mRNA) | uc003mcm.1 |

The invention preferably relates to any combination of the regions according SEQ ID NO. 1, 2 and 3, and SEQ ID NO. 4, 5, 6, and/or 7. For example SEQ ID NO. 1, 2, 3 and 4; SEQ ID NO. 1, 2, 3 and 5; SEQ ID NO. 1, 2, 3 and 6; SEQ ID NO.1, 2, 3 and 7; SEQ ID NO. 1, 2, 3, 4 and 5; SEQ ID NO. 1, 2, 3, 4 and 6; and any other combination may be used, as long as SEQ ID NO. 1, 2, 3 and 4 are present.

Analyzing the methylation status of a genomic region means analyzing the methylation status of at least one CpG position per genomic region.

The inventors surprisingly found that the methylation status within a genomic region according to the invention is almost constant, leading to a uniform distribution methylation levels within said genomic region. In one embodiment of the invention, all CpG positions of a genomic region are analyzed. In a specific embodiment, CpG positions in the vicinity of the genomic region may be analyzed. In an alternative embodiment, a subset of CpG positions of a genomic region is analyzed. Ideally, 1, 2, 3, 4, 5, 6, 7, 8, 9, 10 CpG positions of a genomic region are analyzed. Therefore, a preferred embodiment of the invention relates to a method, wherein analyzing the methylation status of a genomic region means analyzing the methylation status of at least one CpG position per genomic region.

Significantly, the inventors found that a minimum of one genomic region is sufficient to accurately discriminate between aggressive malignant versus non-aggressive malignant as well as benign tissues to be used in prognosis. The extension with additional genomic regions even increases the discriminatory potential of the marker set. Thus, in another embodiment, the invention relates to a method, wherein the methylation status of a further genomic region and/or a further biomarker is analyzed.

In one embodiment of the invention, a known cancer biomarker is additionally analyzed. For PCa these may be, e.g. GSTP1, multidrug resistance protein 1 (MDR1), 0-6-methylguanine-DNA methyltransferase (MGMT), Ras association domain family member 1 (RASSF1), retinoic acid receptor beta (RARB), adenomatous polyposis coli (APC), androgen receptor (AR), cyclin-dependent kinase inhibitor 2A (CDKN2A), E-cadherin (CDH1) and/or CD44. Such biomarkers can also be based on gene expression, e.g. of said encoding genes. In a preferred embodiment, the concentration or activity of prostate specific antigen (PSA) is determined by means of an immunoassay. The analysis of the biomarkers within this context can be the analysis of the methylation status, the analysis of the gene expression (mRNA), or the analysis of the amount or concentration or activity of protein.

For breast cancer these may be typical breast markers such as ER, PR, HER2, proliferation markers such as Ki67, or RNA panels such as OncotypeDx, Mammaprint or PAM-50.

In another embodiment a further genomic region according to the invention is analyzed.

The methylation status is analyzed for example by non-methylation-specific PCR based methods, methylation-based methods or microarray-based methods.

In a preferred embodiment of the invention pyrosequencing may be used for the analysis of the methylation status. epiTYPER or HRM methods may also be preferred as they are relatively simple and quantitative.

As aforementioned the analysis of the DNA methylation status can require genomic region specific primers for the amplification of a sequence in the vicinity of a genomic region.

Furthermore, genomic region specific probes can be required in order to detect the methylation status directly or indirectly. Said nucleic acids may be used in techniques such as quantitative real-time PCR, using for example SYBR®Green, or using TaqMan® or Molecular Beacon techniques, where the nucleic acids are used in the form of genomic region specific primers or genomic region specific probes, such as a TaqMan labelled probe or a Molecular Beacon labelled probe. Within the context of the invention, the nucleic acid selectively hybridizes in the vicinity of the genomic region as defined above. Most preferably it hybridizes selectively within the genomic region of interest. In one embodiment of the invention, a single genomic region specific nucleic acid is used. In a preferred embodiment, the nucleic acids are used as a pair, wherein the first nucleic acid is specific for the sense strand and the second nucleic acid for the antisense strand of the DNA sequence in the vicinity of the genomic region.

Thus, the disclosure relates to a nucleic acid molecule that hybridizes under stringent conditions in the vicinity of one of the genomic regions according to SEQ ID NO. 1 to SEQ ID NO. 7, wherein said vicinity relates to a position as defined herein.

In one embodiment said nucleic acid is 15 to 100 nt in length. In a preferred embodiment said nucleic acid is 15 to 50 nt, in a more preferred embodiment 15 to 40 nt in length.

In one embodiment said nucleic acid is a primer.

Within the context of the invention, genomic region specific primers are used to amplify selectively the DNA sequence in the vicinity of one of the genomic regions by means of PCR as part of the analysis method of the DNA methylation status. Thus, in one embodiment of the present invention the primer is specific for one of the genomic regions and hybridizes in the vicinity, within the meaning specified herein.

The methylation status of a genomic region may be detected directly or indirectly by using a genomic region specific probe. Thus, in one embodiment of the present invention said nucleic acid is a probe which hybridizes in the vicinity of said region. The probe may be methylation site specific.

In a preferred embodiment of the present invention the probe is labelled.

Current methods for the analysis of the methylation status require a bisulphite treatment a priori, thereby converting non-methylated cytosines to uracils. To ensure the hybridization of the genomic region specific nucleic acid of the invention to the bisulphite treated DNA, the nucleotide sequence of the nucleic acid may be adapted. For example, if it is desired to design nucleic acids being specific for a sequence, wherein a cytosine is found to be differentially methylated, that genomic region specific nucleic acid may have two sequences: the first bearing an adenine, the second bearing an guanine at that position which is complementary to the cytosine nucleotide in the sequence of the genomic region. The two forms can be used in an assay to analyze the methylation status of a genomic region such that they are capable of discriminating between methylated and non-methylated cytosines. Depending on the analysis method and the sort of nucleic acid (primer/probe), only one form or both forms of the genomic region specific nucleic acid can be used within the assay. Thus, in an alternative embodiment of the present invention the nucleic acid hybridizes under stringent conditions in said vicinity of one of the genomic regions after a bisulphite treatment. Thus, it may hybridize to a CpG position after bisulphite treatment. In one alternative to the methylated version in the other alternative to the non-methylated version of said position.

The means and methods of the present invention comprise the use of genomic region specific nucleic acids for the diagnosis and/ or prognosis of aggressive cancer, preferably PCa or IBC.

The analysis may also be done by sequencing. Historically there have been two successful approaches to DNA sequence determination: the dideoxy chain termination method, e.g. Sanger et al, Proc. Natl. Acad. Sci., 74: 5463-5467 (1977); and the chemical degradation method, e.g. Maxam et al, Proc. Natl. Acad. Sci. , 74: 560-564 (1977). However, the desire for higher throughputs and more cost-effective sequencing methods has lead to a number of next generation sequencing methods which are for example reviewed in Metzker, Genome Research 15:1767-1776 (2005) and Next-Genration Genome Sequencing, edited by M. Janitz, Wiley-VCH Verlag, Weinheim/Germany, 2008.

Some of the more recent methods are based on a "sequencing by synthesis" approach. Such methods include for instance the pyrosequencing ("454 SequencingTM", Roche Diagnostics) technology which is based on pyrophosphate release, its conversion to ATP and the production of visible light by firefly luciferase (EP 0 932 700 B1; Ronaghi, Genome Research 11:3-11 (2001)). It may be used.

The SOLiDTM ("Sequencing by Oligonucleotide Ligation and Detection") method (Life Technologies; WO 06/084132 A2) is based on the attachment of PCR amplified fragments of template nucleic acids via universal adapter sequences to magnetic beads and subsequent detection of the fragment sequences via ligation of labelled probes to primers hybridized to the adapter sequences. For the readout a set of four fluorescently labeled di-base probes probes are used. After read-out, parts of the probes are cleaved and new cycles of ligation, detection and cleavage are performed. Due two the use of di-base probes, two rounds of sequencing have to be performed for each template sequence. It may also be used and is preferred herein.

Another ligation-based sequencing method is known as CycLiC (Cyclic Ligation and Cleavage). CycLic uses oligonucleotide libraries in which all but one nucleotide is degenerate. The method involves iterative primer extension cycles, base-by-base chain growth by successive ligation and detection steps using labelled oligonucleotides (Mir et al, Nucleic Acids Research 37(1), e5 (2009)). It may be used also.

The Illumina Solexa® sequencing method is based on sequencing-by-synthesis chemistry (Bentley, Curr Opin Genet Dev. 16(6):545-552 (2006)). Large numbers of unique "polonies" (polymerase generated colonies) are generated that can be simultaneously sequenced. These parallel reactions occur on the surface of a "flow cell" which provides a large surface area for many thousands of parallel chemical reactions. It is also preferred.

WO 2007/133831 discloses a method for sequencing a nucleic acid, comprising the step of ligation and an adapter library. The sequencing template is a large concatemeric repeat of a single sequence. This is an option.

US-A1 6 013 445 relates to a further sequencing method.

The nucleic acid for performing the method according to the invention is advantageously formulated in a stable composition. Accordingly, the present invention relates to a composition for the diagnosis and/ or prognosis of aggressive cancer comprising said nucleic acid.

The composition may also include other substances, such as stabilizers, e.g. EDTA, protective nucleic acid carriers etc.

The disclosure also encompasses a kit for the diagnosis and/ or prognosis of aggressive cancer preferably PCa or IBC, comprising the inventive nucleic acid as described above.

The kit may comprise a container for a first set of genomic region specific primers. In a preferred embodiment, the kit may comprise a container for a second set of genomic region specific primers. In a further embodiment, the kit may also comprise a container for a third set of genomic region specific primers. In a further embodiment, the kit may also comprise a container for a forth set of genomic region specific primers, and so forth.

The kit may also comprise a container for bisulphite, which may be used for a bisulphite treatment of the genomic region of interest.

The kit may also comprise genomic region specific probes.

The kit may comprise containers of substances for performing an amplification reaction, such as containers comprising dNTPs (each of the four deoxynucleotides dATP, dCTP, dGTP, and dTTP), buffers and DNA polymerase.

The kit may also comprise nucleic acid template(s) for a positive control and/or negative control reaction and vials with different concentrations of known nucleic acids to allow for construction of a dose-response curve to allow accurate quantitation of the targets. In one embodiment, a polymerase is used to amplify a nucleic acid template in PCR reaction. Other methods of amplification include, but are not limited to, ligase chain reaction (LCR), polynucleotide-specific based amplification (NSBA), or any other method known in the art.

The kit may also comprise containers of substances for performing a sequencing reaction, for example pyrosequencing, such as DNA polymerase, ATP sulfurylase, luciferase, apyrase, the four deoxynucleotide triphosphates (dNTPs) and, e.g. luciferin.

**Table 1: Univariate survival analysis of the four principal risk genes and risk groups resulting from dichotomization at the cut-offs as defined herein into high or low methylation groups; total number of women are indicated by N and number of women with breast cancer recurrence are shown as (Events). P-values are based on the Logrank test (all indicated p values were significant with an alpha of less than 0.05).**

| **Gene** | **Low Methylation N (Events)** | **High Methylation N (Events)** | **N missing (Events)** | **P-value** |
|---|---|---|---|---|
| SFN | 87 (12) | 21 (8) | 15 (5) | 0.007 |
| TWIST1 | 45 (15) | 55 (9) | 10 (1) | 0.024 |
| SERPINB5 | 67 (9) | 42 (13) | 12 (3) | 0.024 |
| SLIT2 | 107 (19) | 12 (6) | 2 (0) | 0.005 |
| | | | | |

### FIGURE CAPTIONS

**Fig. 1****:**
   Methylation values of high PCa Gleason (> 7, triangle) and low Gleason score (<= 7, circle) cases for three pairs of genes. The separating lines show linear discrimination boundaries that were fitted to classify high from low Gleason score. The aim was to separate these two categories as much as possible. The genes plotted are A) SFN and SERPINB5, B) SFN and SLIT2, C) SERPINB5 and SLIT2. A high Gleason score on pathology (regarded as a score of 8 or higher) is a surrogate marker for an aggressive prostate cancer.

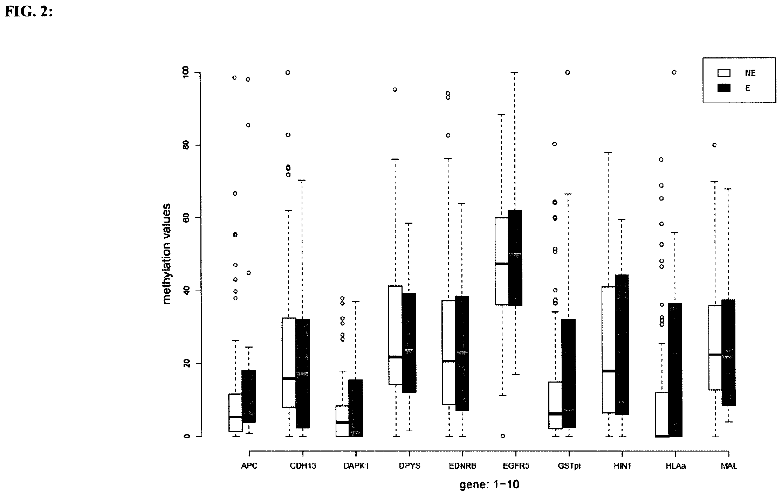

**Fig. 2**: (comparative example)
   A box-whisker plot of differentially methylated genes 1 to 10 showing the range of methylation percentages in a study of 121 women with surgically treated cancer who were followed up for at least 5 years and in whom 25 invasive recurrence events (E) or non-recurrences (N) of the originally treated breast cancers were subsequently recorded. Each lightly shaded vertical box shows the 25 and 75 percentiles of the DNA methylation percentage of the named genes for the non-events N and similarly the darkly shaded box shows the DNA methylation percentages among the events E. The thick horizontal lines in the middle of the boxes show the 50% values. The 5 and 95 percentiles are shown by the horizontal bars on the bottom or the top of the whiskers respectively and individual outlier values are shown as circles below (low outliers) or above (high outliers) the specified ranges.

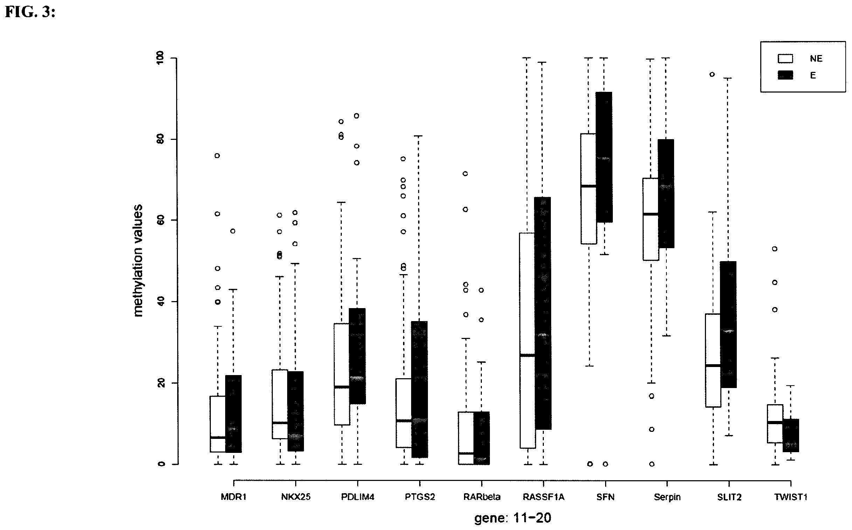

**Fig. 3**: (comparative example)
   Fig 3 shows a box-whisker plot of differentially methylated genes 1.1 to 20 showing the range of methylation percentages in a study of 121 women with surgically treated cancer followed for 5 years.
**Fig. 4**: (comparative example)
   Kaplan Meier curves showing the ability of TWIST1 (a reverse association gene) to separate the 121 women of the breast cancer follow-up cohort into two groups with different risks of recurrence. The women with DNA methylation values above the TWIST1 cut-off (TWIST1+; upper curve) had a lower rate of recurrence than the women below the TWIST1 cut-off (TWIST1-; lower curve). For example at 5 years 68 % of the TWIST- women did not have a recurrence whereas at the same timepoint 83 % of the TWIST1+ women did not have a recurrence. Thus being positive for this marker indicated a 15 % lower absolute risk of IBC recurrence and this difference was significant at the p=0.024 level with the Logrank test.

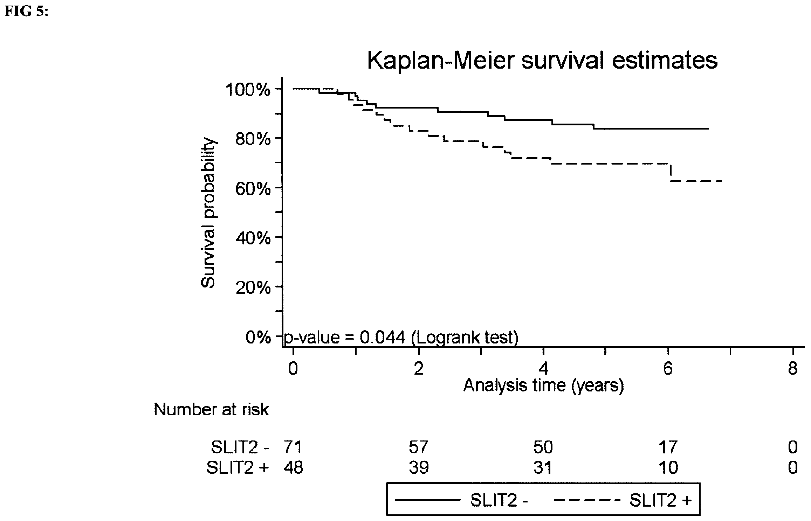

**Fig. 5**: (comparative example)
   Kaplan Meier curves showing the ability of SLIT2 (a forward association gene) to separate the 121 women of the breast cancer follo-wup cohort into two groups with different risks of recurrence. The women with DNA methylation values above the SLIT2 cut-off (SLIT2+; lower curve) had a higher rate of recurrence than the women below the SLIT2 cut-off (SLIT2-; upper curve). For example at 5 years 70 % of the SLIT2+ women did not have a recurrence whereas at the same timepoint 83 % of the SLIT2- women did not have a recurrence. Thus being positive for this marker indicated a 13 % higher absolute risk of IBC recurrence and this difference was significant at the p=0.044 level with the Logrank test.

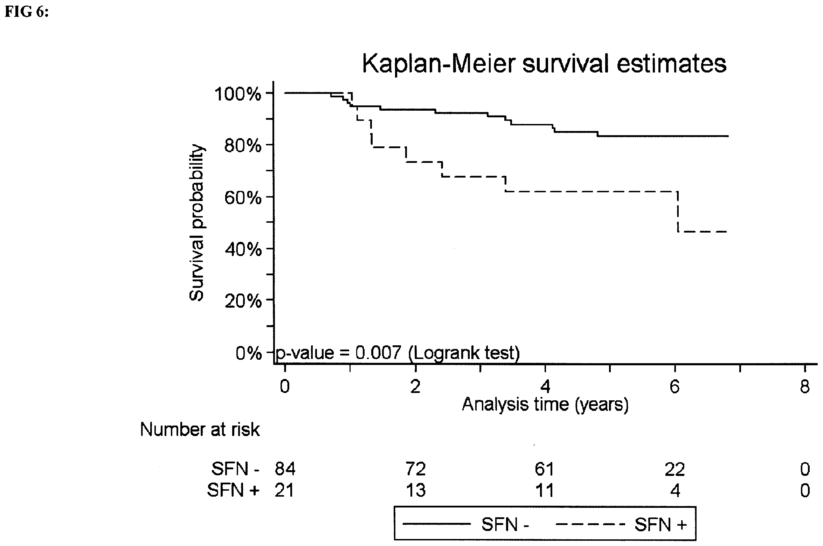

**Fig. 6**: (comparative example)
   Kaplan Meier curves showing the ability of SFN to separate the 121 women of the breast cancer followup cohort into two groups with different risks of recurrence.

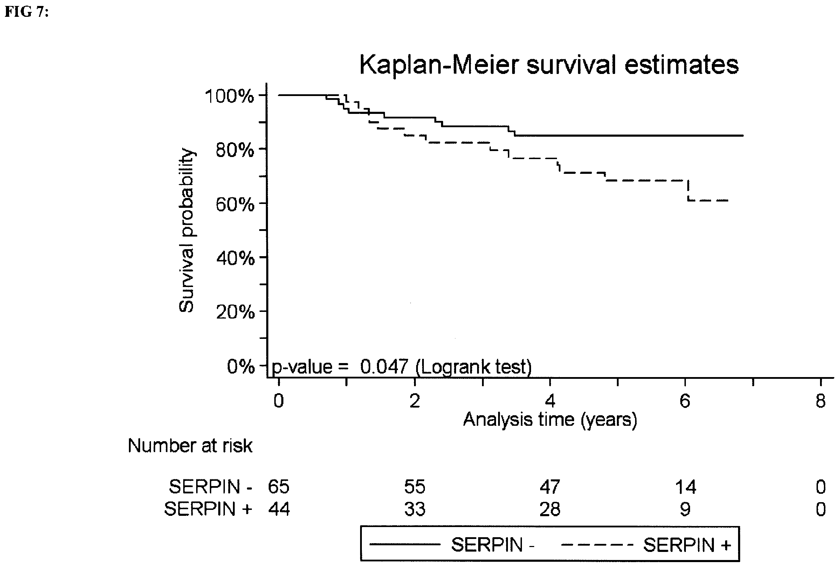

**Fig. 7**: (comparative example)
   Kaplan Meier curves showing the ability of SERPINB5 to separate the 121 women of the breast cancer followup cohort into two groups with different risks of recurrence.

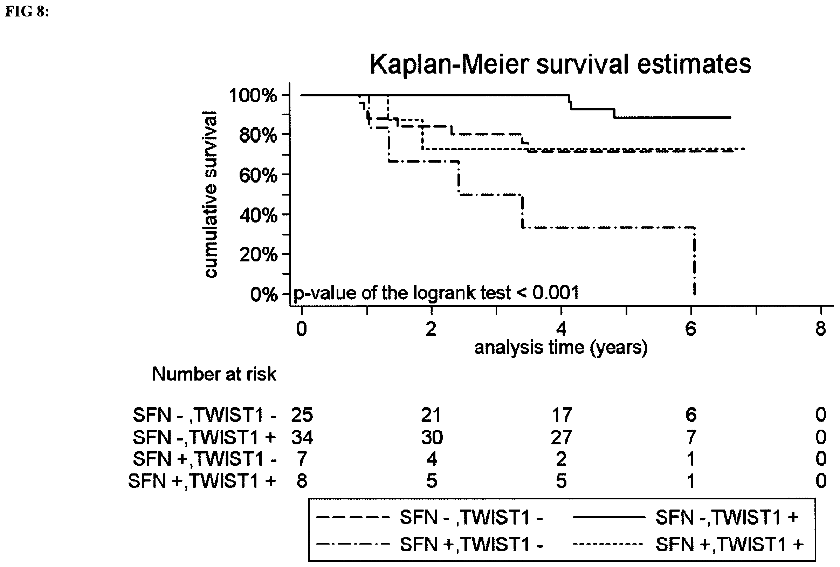

**Fig. 8**: (comparative example)
   Kaplan Meier curves showing the ability of a two-gene combination, SFN and TWIST1 to separate the 121 women of the breast cancer follow-up cohort into two groups with different risks of recurrence. This figure shows the additive (synergistic) effects of combing the two genes. For example at 5 years, of the women who were negative for SFN and positive for TWIST1 (SFN-, TWIST1+), 89 % did not have a recurrence of IBC. In contrast, of women who were positive for SFN and negative for TWIST (SFN+, TWIST1-), 33 % did not have a recurrence of IBC. The absolute difference between these two group is 56 % and the p value for the difference with the Log Rank test was highly significant at p<0.001.

### EXAMPLES

### Human prostate tissue specimens

The study set included fresh frozen prostate tissue from 77 patients of which 48 were diagnosed with cancer and 29 as BPH. Specimens were collected either after radical prostatectomy, transurethral resection of the prostate (TURP) or TURP in cancer patients (channel TURP). Specimens were collected from three different sites, Changhai Hospital in Shanghai, China, Whipps Cross Hospital, London and St Bartholomew's Hospital, London during the period 1996-2008. All specimens were centrally reviewed to confirm diagnosis by expert genitourinary pathologists. Gleason grading was performed by modern standardized criteria.

Informed consent was obtained from all patients. UK national approval was obtained from the Northern Multi-Research Ethics Committee, followed by local ethics committee approval from each of the collaborating hospital trusts. Ethical approval from Changhai Hospital Ethics Committee was obtained for the Chinese specimens.

### DNA extraction and bisulfite conversion

Genomic DNA was extracted from 2-3 10 µm slices of the fresh frozen material using QIAamp DNA Mini Kit (Qiagen Inc., Hilden, Germany) and quantified by UV absorption, typically yielding in total >1 µg of gDNA per specimen. 120-300 ng of gDNA was used in the bisulfite conversion reactions where unmethylated cytosines were converted to uracil with the EpiTect Bisulfite kit (Qiagen) according to manufacturer's instructions. Briefly, DNA was mixed with water, DNA protect buffer and bisulfite mix and the conversion was run in a thermocycler (Biometra, Goettingen, Germany) at the recommended cycle conditions. Converted DNA was purified on a spin column and eluted twice into a total of 40 µl Buffer EB.

PCR and Pyrosequencing: Twenty eight candidate DNA methylation genes were analyzed in the study. Primer sets with one biotin-labelled primer were used to amplify the bisulfite converted DNA. New primers for each of the 28 genes (genes are shown below, gene names follow the UCSC gene nomenclature system http://genome.ucsc.edu/) were designed using PyroMark Assay Design software version 2.0.1.15 (Qiagen); where possible primers were designed to keep amplicons short with lengths between 90 to 140 base pairs (bp) to facilitate later studies of formalin-fixed paraffin-embedded (FFPE) specimens. The size of the amplicons was restricted to a maximum of 210 bp. All primers were located in promoter or first exon CpG islands identified by MethPrimer, depending on where the design of the assay allowed for optimal primers. Due care was taken to avoid any primer overlapping CG dyads to prevent amplification biases. Median size of all amplicons was 104 bp. For genes, previously investigated by other methods, primers were positioned to investigate the same CGs or ones in close vicinity. For some genes e.g. CDH1, GSTP1, we examined two different sites within the CpG island separated by several hundred base pairs. To provide the internal control for total bisulfite conversion, a non-CG cytosine in the region for pyrosequencing was included where possible.

PCRs were performed using a converted gDNA equivalent of 200 cells employing the PyroMark PCR kit (Qiagen). The cell genome-equivalents of DNA calculations assumed 6 pg DNA per diploid cell. Briefly, 12.5 µl master mix, 2.5 µl Coral red, 5pmol of each primer, 7 al of water and 2 µl sample were mixed for each reaction and run at thermal cycling conditions: 95 °C for 15 min and then 45 cycles: 30 sec at 94 °C; 30 sec at the optimized primer-specific annealing temperature; 30 sec at 72 °C and a final extension for 10 min at 72 °C. The amplified DNA was confirmed by electrophoresis in a 2 % low melting point agarose gel (Sigma-Aldrich, Steinheim, Germany) in TBE buffer or by the QiaExel capillary electrophoresis instrument (Qiagen).

A standard pyrosequencing sample preparation protocol was applied. 3 µl streptavidin beads (GE Healthcare, Buckinghamshire, UK), 37 µl PyroMark binding buffer (Qiagen), 20 µl PCR product and 20 µl water were mixed and incubated for 10 min on a shaking table at 1300 rpm. Using the Biotage Q96 Vaccum Workstation, amplicons were separated, denatured, washed and added to 45 µl annealing buffer containing 0.33 µM of pyrosequencing primer. Primer annealing was performed by incubating the samples at 80 °C for 2 min and allowed to cool to room temperature prior to pyrosequencing. PyroGold reagents were used for the pyrosequencing reaction and the signal was analyzed using the PSQ 96MA system (Biotage, Uppsala, Sweden). Target CGs were evaluated by instrument software (PSQ96MA 2.1) which converts the pyrograms to numerical values for peak heights and calculates proportion of methylation at each base as a C/T ratio. All runs contained standard curves, which comprised a range of control methylated DNA (0 %, 25 %, 50 %, 75 %, and 100 %) to allow standardized direct comparisons between different primer sets. For the standard curves a total of 300 ng of unmethylated (Qiagen) and hypermethylated DNA (Millipore, Billerica, MA, USA) were mixed to obtain the different ratios of DNA methylation and then bisulfite converted as described above.

### Statistical Analyses

The main analyses were based on mean values of all CG analyzed. The number of CGs analyzed varied between two to six in each gene as allowed by software-defined parameters. To limit numbers of assays run, and costs, genes that showed no potential in differentiating between BPH and cancer or between low and high Gleason score were investigated in fewer specimens.

Methylation differences between the tissues were examined by Mann-Whitney test. To account for the high number of genes tested on the same data, the Benjamin and Hochberg step-up procedure for controlling false discovery rate (FDR) was applied with FDR of 1 %.

To explore the relationship between gene methylation and age, methylation was normalized by z-scores, where the raw methylation minus the sample mean was divided by the sample standard deviation. Association between methylation and age was explored by Spearman's test, while for methylation versus Gleason score, the Cuzick trend test was used. For cases with a PSA measurement, the association with methylation was using Spearman's rank test. Further, Spearman coefficients, based on rank orderings of raw gene methylation in all cancers, were calculated to explore correlation in methylation between genes. The cut-offs chosen to present true-positive rates (TP, proportion of cancers correctly classified) and false-positive rates (FP, proportion of non-cancers incorrectly flagged) by gene were chosen using the same cost function for all genes - namely, to minimize FP - TP.

To help investigate methylation associated with high Gleason scores, a random forest classification algorithm was applied. Plots were used to inspect the genes identified by the random forest, with classification boundaries added from linear discriminant analyses. Gleason score classification accuracy 95 % confidence intervals (CI) were based on a non-parametric bootstrap method with 1,000 resamples. All statistical calculations were conducted using software R version 2.9.2. Rejection of the null hypothesis was assumed at an α <0.05.

### Descriptive statistics of candidate gene methylation

The reproducibility of the PSQ method was investigated at the outset by measuring methylation of GSTP1 on three separate occasions. The mean methylation difference between highest and lowest reading for the same sample was 7 % for BPH cases, and 13 % for cancers; the Pearson correlation for runs 1 vs. 2 was 0.90 whereas between runs 1 and 3 was 0.97. This concordance was regarded as acceptable and all subsequent data were based on single measurements.

Methylation data were adjusted for primer bias through re-scaling each gene's methylation measurements by the median standard curve obtained for each primer set. The impact of applying these corrections to the genes had small effects on median methylation differences but allowed comparison across different genes. Of the 28 genes studied, methylation of 20 genes: RARB, HIN1, BCL2, GSTP1, CCND2, EGFR5, APC, RASSF1A, MDR1, NKX2-5, CDH13, DPYS, PTGS2, EDNRB, MAL, PDLIM4, SERPINB5, HLAa, ESR1 and TIG1 could distinguish prostate cancer from BPH tissue at FDR of 1 %. Cut-off levels were calculated to evaluate the diagnostic potential of methylation differences. This allowed dichotomization of the data, where a cut-off of 21 % methylation of RARB separated all cancers from BPH with 100 % accuracy, i.e. TP=100 %, FP = 0 %.

In BPH specimens, EGFR5, DPYS, ESR1, MDR1, SERPINB5 and SFN displayed median methylation above 10 % whereas most other genes were unmethylated (median methylation ∼ 2 %). In particular, SERPINB5 and SFN were methylated to approximately 50 % in BPH. Furthermore, SERPINB5 was the only gene with significantly higher methylation in BPH than cancers (p < 0.001). MCAM, CDKN2A, THRB, TWIST1, CDH1 and DAPK1 were methylated below 10 % in both BPH and PCa.

### Association between demographic and clinical covariates and gene methylation

The relationship among gene methylation levels, age, Gleason score and PSA levels were explored in the PCa. There was a positive association of Gleason score with age (p < 0.001) and PSA (p = 0.0013), though no association between PSA and age (p = 0.22).

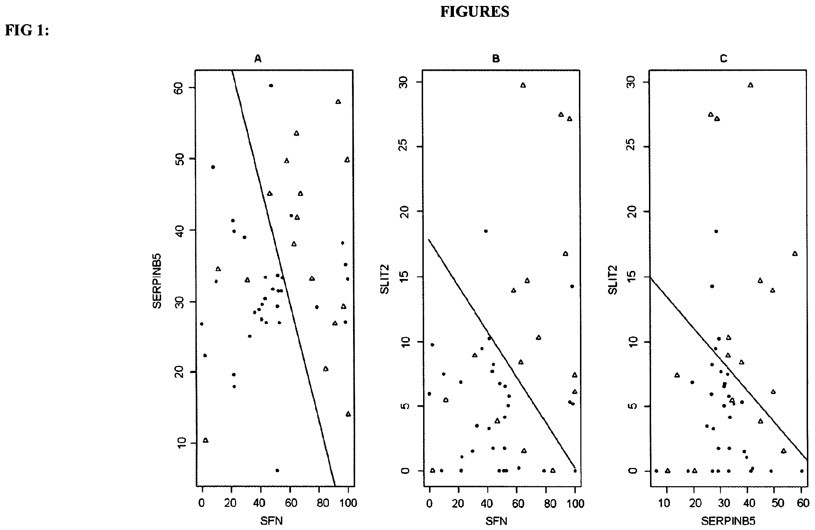

There was a positive trend between age and standardized mean methylation values across all genes, akin to global methylation status, for each case (Pearson correlation 0.52, p < 0.0001). Furthermore, inspection of the distribution of p-values suggested a moderate effect of age common to the methylation of all genes, while Gleason score appeared to affect only subsets of genes. The methylation levels of NKX2-5 and APC (p = 0.009), TIG1, ESR1, GSTP1 (p = 0.01), CDH13, EGFR5 (p = 0.02), MCAM (p = 0.03) and SLIT2 (p = 0.04) showed a positive association with age. The Cuzick trend test showed that the methylation of SFN (p = 0.01), TIG1 (p = 0.02), PDLIM4, APC and SERPINB5 (p = 0.04) were associated with Gleason score . Moreover, according to random forest classification, high methylation of SFN, SLIT2 and SERPINB5 separated low from high Gleason score cancers (Figure 1). The linear discrimination boundaries described the structure found by the random forest classification. Methylation level composite measure of SFN and SERPINB5 correctly classified 81 % (95 % CI 56-91) of high Gleason scores while 23 % (9 - 47) of low Gleason scores were misclassified. Similarly, methylation of SFN and SLIT2, detected 62 % (47-81) of high and misclassified 12 % (9 - 47) low Gleason scores while methylation of SERPINB5 and SLIT2 detected 62 % (47-81) of high and misclassified 13 % (3 - 31) of low Gleason scores. Methylation levels of 17 genes: HIN1, TWIST1, GSTP1, RARB (p < 0.001), HLAa, BCL2, APC, PDLIM4, PTGS2, DPYS, CDH13 (p < 0.01) and RASSF1A, MDR1, EGFR5, EDNRB, TIG1, CCND2 (p < 0.05) were positively associated with PSA. Furthermore, methylation levels of most genes that could distinguish between BPH and cancer e.g. RARB, APC, EGFR5, HIN1, RASSF1A, PTGS2 and CDH13 were moderately correlated.

Other than PSA, with all its limitations, no generally accepted validated biomarkers are currently available for prognosis or therapeutic prediction in prostate cancer. Although several new markers such as PCA3, TMPRSS-ERG, Ki-67, HSP27 and others are under consideration, they are not validated for widespread use and thus Gleason score and PSA in the context of other clinical information remain the mainstay of decision making in PCa.

Twenty of the investigated genes, namely RARB, HIN1, BCL2, GSTP1, CCND2, EGFR5, APC, RASSF1A, MDR1, NKX2-5, CDH13, DPYS, PTGS2, EDNRB, MAL, PDLIM4, SERPINB5, HLAa, ESR1 and TIG1 were more highly methylated in cancers than BPH tissue while the risk of false discovery (FDR) was less than 1 %. To best of our knowledge, the methylation of MAL, HLAa, SERPINB5, THRB, TWIST1 and SLIT2 was demonstrated here for the first time in prostate tissue. While THRB and TWIST1 were overall unmethylated, HLAa and MAL displayed low methylation with fair ability to discriminate between the tissues with median difference 15 % and 7 % respectively. Methylation status of HLAa was associated to level of PSA but not age, Gleason score or methylation of other genes. The methylation of SLIT2 was low, however, median methylation was elevated in cancers ∼6 % vs. ∼ 2 % in BPH and moreover methylation level of SLIT2 could separate high and low Gleason score cancers. Average methylation of SERPINB5 and SFN was lower in cancers than in BPH, 15 % (p < 0.001) and 12 % (p = 0.05) respectively.

MCAM was unmethylated in the investigated specimens despite that the aberrant methylation of the MCAM promoter in PCa was previously reported.

Reports of methylation of DAPK1, CDH1 and CDKN2A have been inconsistent. We observed equally low (median < 10 %) methylation of these genes in BPH and cancer tissues, although non-significant differences of small magnitude were observed. For CDH1, this observation was true for both promoter regions previously reported to show differences in methylation. The inconsistencies may be due to amplification of non-significant differences by a semi-quantitative method resulting in skewed proportions of methylation. In addition, these genes appeared to have little association with Gleason score or PSA levels.

Baseline PSA data were available for only 35 cancers, nonetheless methylation of HIN1 was positively associated with PSA (p < 0.001) with no evidence of a concurrent association with age or Gleason score. Except for TWIST1, 16 of the genes showing difference between cancer and BPH were also positively associated with PSA (p < 0.05).

### Breast cancer patients, pathology specimens and handling (comparative example):

The study included 124 breast cancer patients presenting at Dong-A University Medical Centre, Busan, Republic of Korea from January 2004 to December 2006. All consecutive operable breast cancer patients who consented to provide fresh breast tumour and adjoining normal tissue specimens (measuring approximately 0.5 x 0.5cm each) at the time of surgery and in whom such sample collection and immediate freezing of samples and kept them in -70 deep freezer was possible without jeopardizing clinical diagnosis and management were included in the study. Patients receiving neo-adjuvant chemotherapy (NACT) were included. Patients with missing clinico-pathological data for 2 or more variables were excluded (n = 3), resulting in 121 eligible participants.

All specimens were centrally reviewed to confirm diagnosis by the breast pathologist (DCK). Histopathological evaluation and immunohistochemistry for ER (DAKO, Clone 1D5, 1:50), PgR (DAKO, PgR 636, 1:100) and HER2 (Neomarkers, Clone e2-4001/3B5, Fremont, CA, USA,1:200) were done as per factory recommended protocols, which meet international standards. Histological grade was determined by the Nottingham Modification of Richardson Bloom Score (RBS). HER2 expression was assessed by immunohistochemistry (IHC) and confirmatory HER2 FISH (PathVision^{®} HER2 DNA probe kit, Vysis Inc., Downers Grove, IL, USA) was performed on patients who had 2+ or 3+ (strong membrane staining of over 10 % tumor cells) on IHC, only these confirmed women were considered HER2 positive. Clinico-pathological and treatment variables including age, tumour size (only pathological), type, histologic grade, lymph node status, ER, PgR, HER2, type of surgery, neo-adjuvant / adjuvant treatment details (e.g. type of chemotherapy, no. of cycles, no. of fractions of radiotherapy, tumour bed boost etc.) were recorded in the study database for all eligible patients. Data on tumour size were a mixture of clinical and pathological tumour size, and some patients also received NACT. In view of the heterogeneous data on this variable, all analyses were done excluding the tumour size variable. Patients were regularly followed up every 6 months.

This study was approved by the Institutional Review Board of Dong-A University Medical Centre and all patients gave written informed consent.

A simple macrodissection of tissue slices was performed before DNA extraction to enrich for areas of cancer, the method employed is quick (approximately 10 min per case) and readily mastered by the average laboratory technician. Five consecutive sections per specimen (10 µm thickness) were obtained by cryo-sectioning the cancer tissues and staining the first and fifth sections by H&E for histopathology review to confirm the areas of cancer and to guide the dissections of the three central sections. Genomic DNA was extracted from the three slices of tissue material using QIAamp DNA Mini Kit (Qiagen Inc., Hilden, Germany) and quantified by UV absorption (Nanodrop, Thermo Scientific, Wilmington, Delaware, USA), a majority of sections yielding a combined >1 µl of gDNA per specimen. 120 - 300 ng of gDNA was used in the bisulfite conversion reactions where unmethylated cytosines were converted to uracil with EpiTect Bisulfite kit (Qiagen) according to the manufacturer's instructions. Briefly, DNA was mixed with water, DNA protect buffer and bisulfite mix and the conversion was run in a thermocycler (Biometra, Goettingen, Germany) at the recommended cycle conditions. Converted DNA was purified and eluted in 2 steps into a total 40 µl Buffer EB and further diluted into 20 µl aliquots of 100 cell-equivalents/µl. (the cell calculations assumed 6 pg DNA per diploid cell).

Primer sets with one biotin-labelled primer were used to amplify the bisulfite converted DNA samples. Thirty genes were identified from the literature as candidate genes for this study as previously described. New primers for each of the 30 genes were designed using PyroMark Assay Design software version 2.0.1.15 (Qiagen), with an aim to keep amplicons short with lengths between 90 to 140 base pairs (bp) to facilitate later studies on FFPE specimens. Maximum permissible size of the amplicons was 210 bp. All primers were located in promoter or first exon CpG islands identified by MethPrimer depending on where the design of the assay allowed for optimal primers. CG dyads were not allowed in any forward, reverse or sequencing primer positions to prevent any amplification bias. Mean size of all of the amplicons was 117 bp. For genes, previously investigated by other methods, primers were positioned to investigate the same CGs or ones in close vicinity. To provide the internal control for total bisulfite conversion, a non-CG cytosine in the region for pyrosequencing was included where possible. Three to six CG positions were investigated in each gene.

PCRs were performed using a bisulfite converted gDNA equivalent of 200 to 400 cells employing the PyroMark PCR kit (Qiagen). Briefly, 12.5 µl master mix, 2.5 µl Coral red, 5 pmol of each primer, 7 µl of water and 2 µl sample were mixed for each reaction and run at thermal cycling conditions: 95 °C for 15 min and then 45 cycles: 30 sec at 94 °C; 30 sec at the optimized primer-specific annealing temperature; 30 sec at 72 °C and a final extension for 10 min at 72 °C. The correct amplified DNA was confirmed by electrophoresis in a 2 % low melting point agarose gel (Sigma-Aldrich, Steinheim, Germany) in TBE buffer or by the QiaExel capillary electrophoresis instrument (Qiagen). A standard pyrosequencing sample preparation protocol was applied. 3 µl streptavidin beads (GE Healthcare, UK), 37 µl PyroMark binding buffer (Qiagen), 20 µl PCR product and 20 µl water were mixed and incubated for 10 min on a shaking table at 1300 rpm. Using the Biotage Q96 Vaccum Workstation, amplicons were separated, denatured, washed and added to 45µl annealing buffer containing 0.33 µM of pyrosequencing primer. Primer annealing was performed by incubating the samples at 80 °C for 2 min and allowed to cool to room temperature prior to pyrosequencing. PyroGold reagents were used for the pyrosequencing reaction and the signal was analyzed using the PSQ 96MA system (Biotage, Uppsala, Sweden). Target CGs were evaluated by instrument software (PSQ96MA 2.1) which converts the pyrograms to numerical values for peak heights and calculates proportion of methylation at each base as a C/T ratio. All runs contained standard curves, which comprised a range of control methylated DNA (0 %, 25 %, 50 %, 75 %, and 100 %) to allow standardized direct comparisons between different experiments. For the standard curves a total of 300 ng of unmethylated (Qiagen) and hypermethylated DNA (Millipore, Billerica, MA, USA) were mixed to obtain the different ratios of DNA methylation and then bisulfite converted as described above.

A further selection of preferred genes from the initial 30 candidate genes was performed after the first 30 samples were processed. Genes (n = 20) correlating to (p <0.1) any of Age, Nodal status, Histological grade, ER, PgR, HER2 were selected as preferred. The remaining 10 genes had very low methylation frequency and levels and therefore were unlikely to succeed as biomarkers. These genes were therefore not investigated further in this study; we report findings on 20 selected preferred genes.

All samples were assayed once except for the samples which did not yield pass results on first assay. Such samples were assayed once more and data were recorded as missing if the samples were unsuccessful in the second instance as well. Failed samples constitute a very low proportion of the samples investigated.

The main analyses converted individual C/T ratio data into mean values of all CG analyzed in a particular gene segment. The number of CGs analysed varied between two to six in each gene. Methylation data were adjusted for primer bias through re-scaling each gene's methylation measurements by the median standard curve obtained using control mixtures for each primer set.

Correlation analyses were performed using Spearman's test to investigate associations between various clinico-histopathological variables (categorical) like age, nodal status, grade, ER, PgR, HER2 and percentage methylation (MeC%) of genes (on a continuous scale).

Univariate analyses using log-rank test were performed with recurrence-free survival (RFS) as an endpoint to probe relationship between nodal status, grade, ER, PgR, and HER2. Kaplan-Meier survival estimates were plotted. Various approaches are used for biomarker assessment and outcome-based cut-point optimization, X-Tile is one such approach. Cut-off determination, however, is subjective in the X-Tile approach. We employed a similar approach with objective parameters to determine cut-offs. Different cut-offs from 0 % to 99 % MeC%, in steps of 1 % were used in univariate analyses by log-rank test. Cut-offs yielding one group less than 10 % of whole study population (i.e. <12 subjects) were not considered. The cut-offs yielding lowest p-value were chosen (optimal cut-off by p-value method). In case of multiple cut-offs resulting in the same lowest p-value, a cut-off that resulted in maximum difference in the numbers in two groups was selected (one group below cut-off and other above); essentially lower cut-off with same p-value if equivalent cut-offs are below 50 % and higher cut-off if they are above 50 % were selected.

Cut-offs below 5 % or above 90 % are sensitive to minor differences in assay readings and therefore can result in the incorrect classification of samples. From a reproducibility point of view, it is essential that cut-offs are not affected by minor variations in different experimental runs. Therefore, an additional condition, cut-off >/= 5% and </= 90 %, was applied before genes could be considered for multivariate analyses and other additional analyses; genes with the best cut-off below 5 % or above 90 % were not considered in further analyses in this study.

All statistical calculations were conducted using software R version 2.9.2 or SAS 10.0. All p-values are two-sided with an α < 0.05 unless otherwise specified.

One hundred and twenty-one patients met the inclusion criteria for this study. All patients underwent either a breast conservation surgery or mastectomy. Axillary management was complete axillary clearance or sentinel node biopsy (with complete axillary clearance in whom SNB showed metastatic node/s). Sixteen patients underwent breast conservation surgery, 104 underwent total mastectomy and radical mastectomy was performed in 1 patient. Forty nine patients underwent SNB as the initial axillary staging procedure. Thirteen patients received some NACT, while 99 patients received chemotherapy only post-operatively. Nine patients did not receive any chemotherapy, 46 received methotrexate-based chemotherapy (CMF), 19 received Anthracycline-based chemotherapy (FAC or FEC) and 32 patients received a sequential combination of anthracyclines and taxanes (AC/EC followed by Paclitaxel or Docetaxel). Twenty-three patients additionally received Doxifluridine after completion of standard adjuvant chemotherapy. Sixty patients received radiotherapy (all conservative surgery patients included), 16 of them received tumour bed boost. All premenopausal hormone receptor positive patients were prescribed Tamoxifen for 5 years, while postmenopausal patients were prescribed aromatase inhibitors. After a median follow-up of 5.1 years (4.87-5.4) 25 patients experienced recurrences, while 3 patients died. One patient died due to chemotherapy related complications after first cycle of CMF chemotherapy, cause of death was not know in the other two patients. Patients who died within the 5-year study period were excluded from our biomarker modeling analyses.

Most (n = 112) patients had Invasive Ductal Carcinoma (IDC), 10 of these showed medullary features; 4 patients had Invasive Lobular Carcinoma (ILC), and 5 tumours had other morphologies like mucinous carcinoma or tubular carcinoma.

Correlations among various histopathological factors, between genes and clinico-pathological factors and among genes were investigated. ER showed a strong positive correlation with PR (Spearman's Rho = 0.79, p < 0.001), and negative correlation with HER2 (Rho = -0.172, p < 0.042), and grade (less ER positivity with increasing grade; Rho = -0.361, p < 0.001). PgR expectedly showed similar correlations; HER2 (Rho = -0.252, p = 0.004), grade (Rho = -0.366, p < 0.001). HER2 and grade did not correlate with each other and nodal status did not show correlation with any other variable.

Figure 2 and 3 show box whisker plots of the DNA methylation percentages for each of the 20 genes that exhibited reasonably measurable differential methylation in IBC. None of the genes correlated with age. Only SERPINB5 and PDLIM4 correlated positively with grade and nodal status respectively. HER2 correlated positively with PDLIM4, RARβ and RASSF1A. A majority of correlations were between genes and ER or PgR. ER and PgR correlated positively with CDH13, EDNRB, EGFR5, HIN1, RASSF1A and negatively with RARβ and SERPINB5. ER alone correlated positively with SLIT2. While most genes showed weak to moderate positive correlations, certain negative correlations were observed, particularly of SERPINB5, SFN and TWIST1. SERPINB5 correlated negatively with EGFR5, MAL, SLIT2, HIN1, CDH13, EDNRB and RASSF1A. Interestingly, SFN, SERPINB5 and EGFR5 showed much higher MeC% compared to other genes, while those for DAPK1, HLAa and RARβ were very low.

Univariate survival analysis explored relationships between age, histological grade, nodal status, ER, PgR, HER2 and RFS. Only nodal status (p = 0.004) was significantly associated with RFS. Histological grade showed a trend of association with RFS (p = 0.086). Both variables were considered for multivariate analysis.

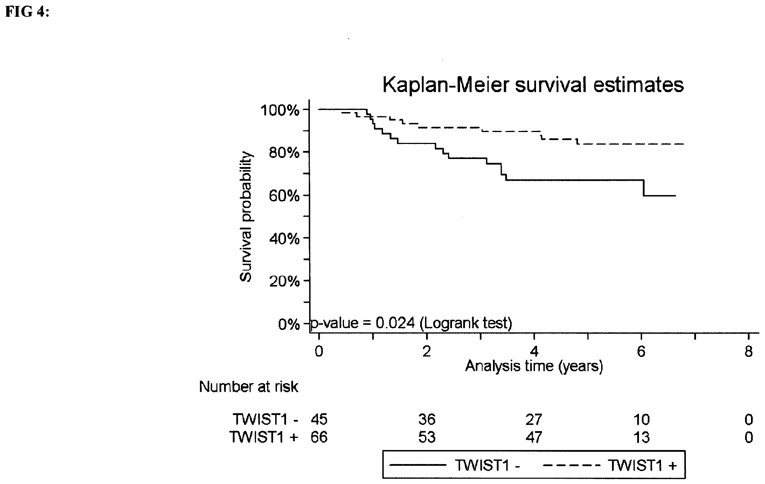

Cut-offs were determined for all 20 genes based on univariate survival analysis using the sliding p-value minimization method described. Eleven genes showed significant association with RFS at chosen cut-offs, 7 genes (HLAa, NKX2-5, APC, SERPINB5, SFN, SLIT2 and TWIST1) were selected for further analyses because they had the most promising statistical characteristics, mainly based on the lowest p values but also in consideration of the robustness of the data in the PSQ assay. Kaplan-Meier survival plots that show the recurrence rates of IBC in different groups of women are displayed for TWIST1 SLIT2, SFN, SERPINB5 and a combination of SFN and TWIST1 in Figures 4, 5, 6, 7, and 8 respectively.

## Claims

1. A method for prognosis of prostate cancer, comprising the steps of analyzing in a sample of a subject the DNA methylation status of the following genomic regions,
(i) SFN according to SEQ ID NO. 1,
(ii) SLIT2 according to SEQ ID NO. 2 and
(iii) SERPINB5 according to SEQ ID NO. 3, and
wherein, if at least two of the following features apply, the sample is categorized as a sample from a patient with cancer with a poor prognosis:
(i) SFN shows a methylation cut-off value of above 80 % and/or,
(ii) SLIT2 shows a methylation cut-off value of above 45 % and/or,
(iii) SERPINB5 shows a methylation cut-off value of above 70 %.

2. A method according to any of the preceding claims, wherein the methylation status of a further genomic region and/or a further biomarker is analyzed which may be selected from the group of
i. APC according to SEQ ID NO. 5
ii. HLAa according to SEQ ID NO. 6 and/or
iii. NKX2-5 according to SEQ ID NO. 7.

3. A method according to any of the preceding claims, wherein analyzing the methylation status of a genomic region means analyzing the methylation status of at least one CpG position per genomic region.

4. A method according to any of the preceding claims, wherein the methylation status is analyzed by non-methylation-specific PCR based methods, methylation-based methods, next generation sequencing or microarray-based methods.

5. A method according to claim 4, wherein the non-methylation-specific PCR based method is pyrosequencing.

6. *In vitro* use of a nucleic acid that hybridizes under stringent conditions in the vicinity of one of the genomic regions according to SEQ ID NO. 1 to SEQ ID NO. 7, wherein said vicinity is any position having a distance of up to 1000 nt from the 3'- or 5'-end of said genomic region and wherein said vicinity includes the genomic region itself for the prognosis of prostate cancer according to the method of claim 1.

7. Use according to claim 6, wherein the nucleic acid is 15 to 100 nt in length.

8. Use according to claims 6 or 7, wherein the nucleic acid is a primer.

9. Use according to claim 8, wherein the primer is specific for one of the genomic regions of claim 1.

10. Use according to claim 6 or 7, wherein the nucleic acid is a probe.

11. Use according to claim 10, wherein the probe is labelled.

12. Use according to any of the claims 6 to 11, wherein the nucleic acid hybridizes under stringent conditions in said vicinity of one of the genomic regions after a bisulphite treatment of the genomic region.

## Patentansprüche

1. Eine Methode zur Prognose von Prostatakrebs, umfassend der Schritte der Analyse des DNA Methylierungsstatus der folgenden genomischen Regionen, in einer Probe eines Subjektes:
(i) SNF gemäß SEQ ID NO. 1,
(ii) SLIT2 gemäß SEQ ID NO.2 und
(iii) SERPINB5 gemäß SEQ ID NO. 3, und
wobei, wenn mindestens zwei der folgenden Merkmale zutreffen, die Probe als eine Probe eines Patienten mit Krebs mit einer schlechten Prognose eingeordnet wird:
(i) SFN zeigt einen Methylierungs-Cut-Off Wert von über 80% und/oder,
(ii) SLIT2 zeigt einen Methylierungs-Cut-Off Wert von über 45% und/oder,
(iii) SERPINB5 zeigt einen Methylierungs-Cut-Off Wert von über 70%.

2. Eine Methode nach einem der vorherigen Ansprüche, wobei der Methylierungsstatus einer weiteren genomischen Region und/oder eines weiteren Biomarkers analysiert wird, welcher ausgewählt ist aus der Gruppe von:
i. APC gemäß SEQ ID NO. 5
ii. HLAa gemäß SEQ ID NO. 6 und/oder
iii. NKX2-5 gemäß SEQ ID NO. 7.

3. Eine Methode nach einem der vorherigen Ansprüche, wobei die Analyse des Methylierungsstatus einer genomischen Region, die Analyse des Methylierungsstatus von mindestens einer CpG Position pro genomischer Region bedeutet.

4. Eine Methode nach einem der vorherigen Ansprüche, wobei der Methylierungsstatus durch Nicht-Methylierungs-spezifische PCR-basierte Methoden, Methylierungs-basierte Methoden, Sequenzierung der nächsten Generation oder Microarray-basierte Methoden, analysiert wird.

5. Eine Methode nach Anspruch 4, wobei die Nicht-Methylierungs-spezifische PCR-basierte Methode Pyrosequenzierung ist.

6. *In vitro* Verwendung einer Nukleinsäure, die unter stringenten Bedingungen in der Nähe von einer der genomischen Regionen gemäß SEQ ID NO. 1 bis SEQ ID NO. 7 hybridisiert, wobei besagte Nähe jede Position ist, die einen Abstand von bis zu 1000 Nukleotiden vom 3'- oder 5' Ende von besagter genomischer Region hat und wobei besagte Nähe die genomischen Region selber beinhaltet, für die Prognose von Prostatakrebs gemäß der Methode von Anspruch 1.

7. Verwendung nach Anspruch 6, wobei die Nukleinsäure 15 bis 100 Nukleotide lang ist.

8. Verwendung nach den Ansprüchen 6 oder 7, wobei die Nukleinsäure ein Primer ist.

9. Verwendung nach Anspruch 8, wobei der Primer spezifisch für eine der genomischen Regionen von Anspruch 1 ist.

10. Verwendung nach den Ansprüchen 6 oder 7, wobei die Nukleinsäure eine Sonde ist.

11. Verwendung nach Anspruch 10, wobei die Sonde markiert ist.

12. Verwendung nach einem der Ansprüche 6 bis 11, wobei die Nukleinsäure unter stringenten Bedingungen in besagter Nähe von einer der genomischen Regionen, nach Behandlung der genomischen Region mit Bisulfit, hybridisiert.

## Revendications

1. Procédé de pronostic du cancer de la prostate, comprenant les étapes de l'analyse, dans un échantillon provenant d'un sujet, de l'état de méthylation de l'ADN des régions génomiques suivantes :
(i) SFN selon SEQ ID NO.1,
(ii) SLIT2 selon SEQ ID NO. 2 et
(iii) SERPINBS selon SEQ ID NO. 3, et
où, si au moins deux des conditions suivantes sont remplies, l'échantillon est classé comme un échantillon provenant d'un patient atteint d'un cancer ayant un mauvais pronostic :
(i) SFN présente une valeur de méthylation seuil au-dessus de 80 % et/ou,
(ii) SLIT2 présente une valeur de méthylation seuil au-dessus de 45 % et/ou,
(iii) SERPINB5 présente une valeur de méthylation seuil au-dessus de 70 %.

2. Le procédé selon l'une quelconque des revendications précédentes, dans lequel l'état de méthylation d'une région génomique supplémentaire et/ou d'un biomarqueur est analysé, qui peut être choisi du groupe de
i. APC selon SEQ ID NO. 5
ii. HLAa selon SEQ DI NO. 6 et/ou
iii. NKX2-5 selon SEQ ID NO. 7.

3. Le procédé selon l'une quelconque des revendications précédentes, dans lequel analyser l'état de méthylation d'une région génomique signifie analyser l'état de méthylation d'au moins une position CpG par région génomique.

4. Le procédé selon l'une quelconque des revendications précédentes, dans lequel l'état de méthylation est analysé par des procédés basés sur la PCR non-méthylation spécifique, des procédés basés sur la méthylation, le séquençage de nouvelle génération ou des procédés basés sur les microarrays.

5. Le procédé selon la revendication 4, dans lequel le procédé basé sur la PCR non-méthylation spécifique est le pyroséquençage.

6. Utilisation *in vitro* d'un acide nucléique qui s'hybride dans des conditions strictes au voisinage d'une des régions génomiques selon SEQ ID NO. 1 à SEQ ID NO. 7, où ledit voisinage est une position ayant une distance de jusqu'à 1000 nt de l'extrémité 3' ou 5' de ladite région génomique et où ledit voisinage comprend la région génomique elle-même, pour le pronostic du cancer de la prostate selon le procédé de la revendication 1.

7. L'utilisation selon la revendication 6, dans laquelle l'acide nucléique a une longueur de 15 à 100 nt.

8. L'utilisation selon l'une quelconque des revendications 6 ou 7, dans laquelle l'acide nucléique est une amorce.

9. L'utilisation selon la revendication 8, dans laquelle l'amorce est spécifique pour l'une des régions génomiques de la revendication 1.

10. L'utilisation selon l'une quelconque des revendications 6 ou 7, dans laquelle l'acide nucléique est une sonde.

11. L'utilisation selon la revendication 10, dans laquelle la sonde est marquée.

12. L'utilisation selon l'une quelconque des revendications 6 à 11, dans laquelle l'acide nucléique s'hybride dans des conditions strictes dans ledit voisinage d'une des régions génomiques après le traitement de la région génomique au bisulfite.
